# EUROPÄISCHE PATENTANMELDUNG

(11) **EP 1 067 377 A2**
(43) Veröffentlichungstag der Anmeldung: **10.01.2001**
(21) Anmeldenummer: 00111292.9
(22) Anmeldetag: 25.05.2000
(51) Int. Cl.: G01N 27/12, G01N 33/00

(54) **Gassensor**

(30) Priorität: 23.06.1999 DE 19928800
(71) Anmelder: SIEMENS AKTIENGESELLSCHAFT, 80333 München (DE)
(72) Erfinder: Weh, Thomas, 81541 München (DE); Fleischer, Maximilian, Dr., 85635 Höhenkirchen (DE); Frank, Joachim, Dr., 85521 Ottobrunn (DE); Meixner, Hans, Prof., 85540 Haar (DE)

(57) **Zusammenfassung**

Der Gassensor weist
eine gassensitive Detektionsschicht (1) auf und ist **dadurch gekennzeichnet**, daß auf der Detektionsschicht (1) mindestens eine Filterschicht (2) und auf der mindestens einen Filterschicht (2) mindestens eine gasaktive Beschichtung (3) vorhanden ist.

## Beschreibung

Die Erfindung betrifft einen Gassensor.

Es besteht eine steigende Nachfrage nach Gassensoren, welche sehr selektiv eine spezielle Gaskomponente eines Gasgemischs detektieren können. Gleichzeitig besteht eine Forderung nach einer Minimierung der Herstellungskosten eines Gassensors. Bei der Realisierung eines Gassensors muß dabei oft ein Kompromiß zwischen niedrigen Herstellungskosten und einer hohen Leistungsfähigkeit eingegangen werden, speziell in Hinsicht auf die Selektivität des Gassensors.

Beispielsweise gestaltet sich die selektive Detektion einer Gruppe von Gaskomponenten aus der Stoffamilie der Kohlenwasserstoffe äußerst schwierig, weil erstens diese Stoffamilie viele Einzelstoffe umfaßt und zweitens die einzelnen Stoffe in ihren chemischen Reaktionen ähnlich sind, insbesondere unter den leicht flüchtigen Einzelstoffen.
Innerhalb dieser Stoffgruppe nehmen Alkohole eine Sonderstellung ein, da für den Nachweis eines Alkohols großes Interesse besteht. Eine typische Applikation, die einen preiswerten und robusten Nachweis eines oder mehrerer Alkohole erfordert, ist die Messung des Alkoholisierungszustandes einer Person, der Nachweis einzuhaltender Ethanol-Grenzwerte innerhalb eines Arbeitsraums oder eine Überwachung eines Gährungsprozesses, z. B. durch eine Membran hindurch.

Zur Detektion eines Gases mit hinreichender Selektivität können beispielsweise Analysegeräte wie Spektrometer oder Gaschromatographen eingesetzt werden, welche sehr teuer und nicht für kontinuierliche Messungen geeignet sind.

Auch kann ein Sensor mit physikalischen Prinzipien eingesetzt werden, insbesondere zum Nachweis von Alkohol, z. B. auf der Basis einer Infrarot-Detektion mit Bandpaßfiltern. Ein solcher physikalischer Sensor besitzt jedoch oft eine große Querempfindlichkeit auf andere Gaskomponenten, wie auf andere Kohlenwasserstoffe, so daß ein selektiver Nachweis, insbesondere von Alkoholen unter einer realistischen Bedingung nicht garantiert werden kann.

Eine weitere Möglichkeit besteht darin, den Einsatzort eines bekannten querempfindlichen Gassensors derart einzuschränken, daß seine Querempfindlichkeit nicht zum Tragen kommt. Eine solche mangelnde Querempfindlichkeit kann in den meisten Fällen selbstverständlich nicht garantiert werden.

In EP 0 464 243 wird ein Sauerstoffsensor mit halbleitendem Galliumoxid beschrieben, bei dem ein gassensitiver Ga₂O₃-Dünnfilm auf einem Al₂O₃-Substrat aufgebracht wird.

In EP 0 464 244 wird ein Sensor zur Erfassung reduzierender Gase offenbart, bei dem das gassensitive Material aus Ga₂O₃-Keramik besteht, und dessen Betriebstemperatur unter 700 °C, vorzugsweise im Bereich 550°C bis 600°C, liegt

Aus EP 0 806 656 sind ein Gassensor und ein Verfahren zur Herstellung desselben bekannt, bei dem zur Erhöhung der Selektivität und der Sensitivität die gassensitive Ga₂O₃-Schicht mit einer Filterschicht, die SiO₂ aufweist, bedeckt ist.

In DE 196 18 705 C1 wird ein Gassensor auf der Basis von Ga₂O₃ zur CH₄-Detektion beschrieben. Dabei wird eine Querempfindlichkeit des Gassensors mittels des verwendeten Auswerte-Algorithmus reduziert.

Es ist eine Aufgabe der vorliegenden Erfindung, einen Gassensor mit geringer Querempfindlichkeit bereitzustellen.

Es ist eine weitere Aufgabe der vorliegenden Erfindung, einen Gassensor mit geringer Querempfindlichkeit bereitzustellen, welcher zudem preiswert herstellbar ist.

Diese Aufgabe wird mittels eines Gassensors gemäß des Patentanspruchs 1 gelöst. Vorteilhafte Ausgestaltungen sind den Unteransprüchen entnehmbar.

Der Gassensor basiert auf einer gassensitiven Detektionsschicht, an der ein Sensorsignal, z. B. ein elektrischer Widerstand, meßbar ist, welches sich in Abhängigkeit von einer Konzentration eines oder mehrerer Gaskomponenten ändern kann. Auf der Detektionsschicht ist mindestens eine Filterschicht und auf der mindestens einen Filterschicht mindestens eine gasaktive Beschichtung vorhanden. Die gasaktive Beschichtung kann eine durchgehend bedeckende gasaktive Schicht sein, aber auch eine nichtdurchgängig bedeckende Beschichtung, z. B. aus Metall-Clustern, die inselförmig auf der Filterschicht aufliegen.

Im folgenden wird zur Vereinfachung eine Gaskomponente eines Gasgemisches als Gas bezeichnet.

Ein Gasgemisch, das aus dem zu detektierenden Gas und anderen, oft in beträchtlichen Mengen vorhandenen Störgasen besteht, trifft am Gassensor auf die gasaktive Beschichtung. Die Wirkung der gasaktiven Beschichtung besteht im wesentlichen in einer Entfernung der Störgase. Dies geschieht beispielsweise durch elektrostatische, katalytische oder chemische Effekte oder durch eine Kombination dieser Effekte.

Bei einer Wirkung als elektrostatischer Filter hält eine positiv geladene Oberfläche der gasaktiven Beschichtung positiv geladene Ionen ab, z. B. mittels p-Halbleitung. Eine solche gasaktive Beschichtung besteht aus p-leitendem SrTiO₃, welche H⁺-Ionen, die z.B. durch Cracken von Kohlenwasserstoffen entstehen, abhält.

Die Wirkung der gasaktiven Beschichtung über den Feldeffekt besteht darin, daß eine elektrische Aufladung der gasaktiven Beschichtung, z. B. aus inselförmig oder durchgehend aufgetragenen Goldclustern, durch eine elektrisch isolierende Filterschicht, z. B. aus SiO₂, hindurch auf die Detektionsschicht, z. B. aus Ga₂O₃, wirkt. In der Detektionsschicht wird die Ladungsdichte verändert, was z.B. durch eine Widerstandsmessung feststellbar ist.
Eine gasaktive Beschichtung aus Goldclustern kann beispielsweise bei einer Adsorption von CO-Molekülen elektrisch geladen werden. Die isolatorischen und elektrischen Eigenschaften der Filterschicht werden zweckmäßigerweise gezielt zur Bildung eines gassensitiven Aufbaus herangezogen werden.

Bei einer Wirkung der gasaktiven Beschichtung mittels katalytischer Umsetzung von Störgasen wird der störende Einfluß verschiedener Störgase durch eine Umsetzung in nicht detektierbare Gase beseitigt.

Zum einfacheren Aufbau und zur einfacheren Aufwertung ist es günstig, wenn zwischen der gasaktiven Beschichtung und dem zu detektierenden Gas keine Wechselwirkung stattfindet.

Zur weiteren Ausweitung einer Anwendungsmöglichkeit kann die gasaktive Beschichtung auf das zu detektierende Gas auch so wirken, daß sie das zu detektierende Gas in eine andere chemische Form bringt, welche mit einem geringeren Aufwand detektierbar ist und welches in einem direkten Zusammenhang mit der Konzentration des zu detektierenden Gases steht.

Es ist zur verbesserten Störgasunterdrückung vorteilhaft, falls mehrere gasaktive Beschichtungen vorhanden sind, und zwar direkt übereinander aufgebracht oder durch Filterschichten getrennt. Die Verwendung der gasaktiven Beschichtungen ist in der Regel vom Anwendungsfall abhängig. Verschiedene gasaktive Beschichtungen können auf verschiedene Gas unterschiedlich wirken und so ein gezielte Selektivität des Gassensors verbessern. Allerdings erhöht die Aufbringung mehrerer Beschichtungen auch den Produktionsaufwand.

Es ist zur Filterung positiv geladener Ionen vorteilhaft, falls die gasaktive Beschichtung p-dotiert ist. Es wird eine gasaktive Beschichtung bevorzugt, welche Cr-dotiertes SrTiO₃ enthält. Cr-dotiertes SrTiO₃ besitzt den Vorteil, daß es in einem Temperaturbereich zwischen 500°C und 1000°C p-leitend und somit zur Anwendung an einem , z.B. halbleitenden, Hochtemperatur-Gassensor geeignet ist.

Zur Detektion von Ethanol wird sowohl eine Ga₂O₃-Dünnschicht als auch eine Al₂O₃-Schicht als gasaktive Beschichtung bevorzugt.

Auch wird eine gasaktive Beschichtung aus Goldclustern bevorzugt, insbesondere zur CO-Detektion.

Die zwischen der gasaktiven Beschichtung und der Detektionsschicht angebrachte Filterschicht hält idealerweise alle unerwünschten Substanzen, die durch die gasaktive Beschichtung nicht abgehalten werden können oder durch diese produziert werden, von der Detektionsschicht ab. Sie sollte vorteilhafterweise dicht sowie chemisch und thermisch stabil sein. Zudem sollte sie vorteilhafterweise elektrisch schlecht leitend sein, um die Leitfähigkeit der gassensitiven Detektionsschicht nicht zu beeinträchtigen.

Die Filterschicht kann beispielsweise SiO₂, Si₃N₄, AlN oder Al₂O₃ enthalten oder auch eine andere keramische Substanz. Selbstverständlich ist die Filterschicht nicht auf eine keramische Substanz beschränkt.

Besonders vorteilhaft ist die Verwendung einer SiO₂-Schicht, insbesondere in Verbindung mit einer gasaktiven p-leitenden Cr-dotierten SrTiO₃-Schicht zur Wasserstoff-Detektion, mit einer Ga₂O₃-Dünnschicht zur Ethanol-Detektion oder mit einer gasaktiven Schicht aus Al₂O₃ zur Alkohol-Detektion.

Es können mehrere, z. B. unterschiedliche, Filterschichten verwendet werden, was eine Vorteil in Bezug auf die Filtermöglichkeiten und -leistung ergibt. Allerdings ist eine solche Lösung vergleichsweise teuer.

Auch ist eine Mehrfachkombination von gasaktiven Beschichtungen und Filterschichten möglich, wobei die Reihenfolge der Schichten bzw. Beschichtungen sich aus dem Anwendungszweck ergibt. Dadurch ist eine noch höhere Selektivität realisierbar ist. Beispielsweise können mehrere Paare einer Filterschicht und einer gasaktiven Beschichtung übereinander angeordnet werden. Dabei können gasaktive Beschichtungen und/oder Filterschichten aus unterschiedlichen Materialien verwendet werden. Auch sind mehrere gasaktive Beschichtungen und/oder Filterschichten übereinander aufbringbar.

Als Detektionsschicht wird eine Beschichtung bevorzugt, welche ein halbleitendes Metalloxid, bevorzugt Ga₂O₃, beinhaltet. Die Detektionsschicht kann beispielsweise auf ihrer der Filterschicht abgewandten Seite mit einer Interdigitalstruktur zur Meßwertaufnahme, beispielsweise in Form einer Widerstandsmessung ausgerüstet sein. Typischerweise ist die Ga₂O₃-Schicht auf einem beheizbaren Substrat aufgebracht und wird bei T ≥ 350°C, meist zwischen 350°C und 950°C betrieben.

In den folgenden Ausführungsbeispielen wird der Gassensor schematisch näher dargestellt.
- Figur 1: zeigt einen Aufbau eines selektiven Gassensors,
- Figur 2a: zeigt eine Meßstruktur, welche auf dem Substrat aufgebracht ist,
- Figur 2b: zeigt eine Heizstruktur des Substrats,
- Figur 3: zeigt als Schnittdarstellung in Seitenansicht einen Aufbau des Gassensors,
- Figur 4: zeigt ein Sensorsignal eines Gassensors in Abhängigkeit eines am Gassensor befindlichen Gases,
- Figur 5: zeigt die Reaktion eines weiteren Gassensors auf seine Beaufschlagung mit verschiedenen Gasen,
- Die Figuren 6 und 7: zeigen die Reaktion des Sensorsignals eines weiteren Gassensors bei Beaufschlagung mit verschiedenen Gasen.

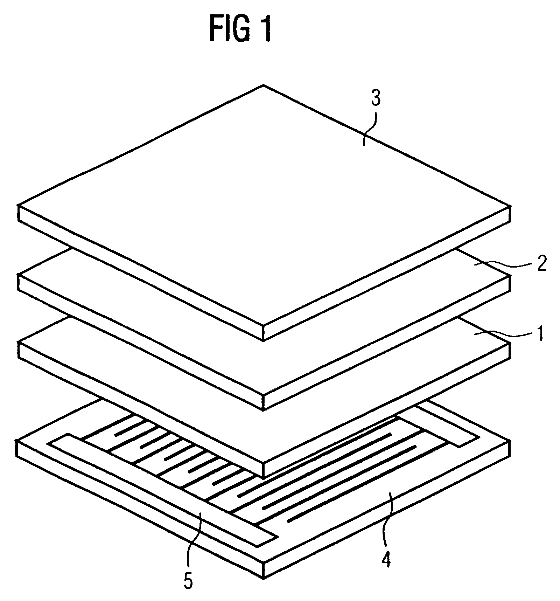

In Figur 1 ist in Schrägansicht in Explosions-Darstellung ein gasselektiver Gassensor gezeigt.

Auf einem Substrat 4 ist ein Meßwertaufnehmer 5 in Form einer Interdigitalstruktur zur Aufnahme eines elektrischen Widerstands R angebracht. Auf der mit der Meßstruktur ausgestatteten Seite des Substrats 4 ist eine Detektionsschicht 1 aufgebracht. Über, d. h. an der dem Substrat 4 abgewandten Seite, der Detektionsschicht 1, ist eine Filterschicht 2 aufgebracht. Auf der Filterschicht 2 wiederum liegt eine gasaktive Beschichtung 3.

Das Substrat 4 ist beheizbar, beispielsweise mittels einer Heizstruktur 6, welche auf der der Meßstruktur 5 abgewandten Seite des Substrats 4 angebracht ist.

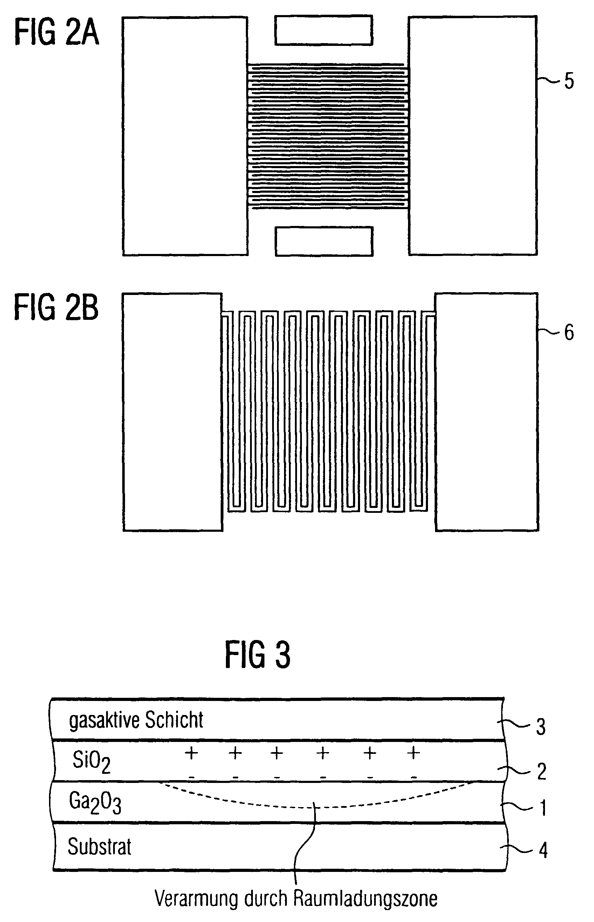

Figur 2a zeigt in Aufsicht die Meßstruktur 5, welche als Interdigitalstruktur ausgebildet ist und über die ein Widerstand R der Detektionsschicht 1 gemessen wird. Der Widerstand R wird durch elektrische Anschlüsse an den hier links und rechts vorhandenen flächigen Teilen abgegriffen.

Figur 2b zeigt in Aufsicht eine mäanderförmige Heizstruktur 6, mittels der das Substrat 4 aufheizbar ist. Dazu muß das Substrat 4 weitestgehend elektrisch isolierend sein. Die Verwendung von Substraten 4 aus SiO₂ (z. B. polykristallin oder als Quarz), Al₂O₃ und Si₃N₄ ist weit verbreitet. Eine typische Betriebstemperatur T für einen Gassensor mit einer Detektionsschicht 1 aus halbleitendem Metalloxid beträgt 300°C bis 1200°C, typischerweise 350°C bis 950°C.

Figur 3 zeigt als Schnittdarstellung in Seitenansicht einen Gassensor mit einer aus Gold-Clustern aufgebauten gasaktiven Beschichtung 3, einer Filterschicht 2 aus SiO₂ und einer beheizten Detektionsschicht 1 aus Ga₂O₃.

Dies entspricht einem herkömmlichen Hochtemperatur-Metalloxidsensor, welcher mit einer zusätzlichen Filterschicht 2 und einer gasaktiven Beschichtung 3 ausgerüstet ist. Der herkömmliche Hochtemperatur-Metalloxidsensor ist ein meßtechnisch einfach auszuwertender Sensor, der sich außerdem durch Robustheit und niedrige Produktionskosten auszeichnet, aber sehr querempfindlich ist.

In diesem Ausführungsbeispiel wird die Wirkung der gasaktiven Beschichtung 3 über den Feldeffekt dargestellt.
Die gasaktive Beschichtung 3, wird durch das zu detektierende Gas, hier: CO, elektrisch aufgeladen. Innerhalb der elektrisch isolierenden Filterschicht 2 kommt es dadurch zu einer teilweisen Trennung von Raumladungen, wobei ihr an die gasaktive Beschichtung 3 grenzender Bereich vorwiegend positiv und ihr an die Detektionsschicht 1 grenzender Bereich vorwiegend negativ geladen ist. Aufgrund dieser Ladungstrennung kommt es innerhalb der Detektionsschicht 1 zu einer Veränderung der Trägerladungsdichte durch eine durch die Raumladungszone hervorgerufenen Verarmung. Die Veränderung der Trägerladungsdichte in der Detektionsschicht 1 läßt sich durch eine über die Meßstruktur 5 durchgeführte Widerstandsmessung feststellen.

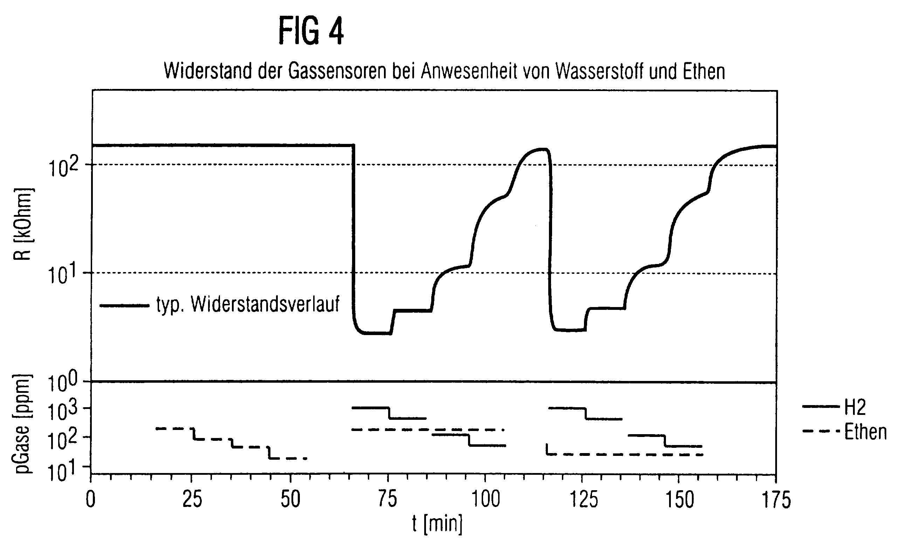

In Figur 4 wird die Reaktion eines Gassensors auf eine Beaufschlagung mit Wasserstoff (H₂)und Ethen gezeigt. Das Sensorsignal ist als Widerstand R in kOhm in logarithmischer Skala aufgetragen gegen die Zeit t in min. Darunter ist auf der gleichen Zeitskala die Gaskonzentration p in ppm (= parts per million) des beaufschlagten Gases, nämlich H₂ (durchgezogene Linie) und Ethen (gestrichelte Linie) aufgetragen.

Der bei dieser Messung verwendete Sensor ist ein selektiver Wasserstoff-Sensor. Er weist als gasaktive Beschichtung 3 p-leitendes, Cr-dotiertes SrTiO₃ und als Filterschicht 2 eine SiO₂-Schicht auf.
Die Wirkung des SrTiO₃ besteht hauptsächlich darin, daß durch seine positiv geladene Oberfläche eine Raumladung entsteht, welche positiv geladene Ionen abhält und das H₂ ungehindert durchläßt. Der Gassensor wird günstigerweise bei einer Temperatur T = 700°C betrieben, wodurch bei einer gleichzeitigen Aufgabe von H₂ und Ethen Crackprodukte der Kohlenwasserstoffe, vor allem H⁺-Ionen, wirkungsvoll von der Detektionsschicht 1 abgehalten werden. Diese Störgase würden sonst das Meßsignal des Gassensors in einem hohen Maß verfälschen.

Die SiO₂-Filterschicht 2 wirkt in diesem Fall als physikalischer Filter, der nur eine selektive Diffusion von H₂ zuläßt. Ohne die gasaktive Beschichtung 3 würde man neben H₂ auch Crackprodukte an der SiO₂-Schicht vorfinden, welche die Funktion dieser Filterschicht 2 erheblich stören könnten. Diese Crackprodukte werden aber durch die gasaktive Beschichtung 3 wirkungsvoll ferngehalten, so daß eine hochselektive Detektion von Wasserstoff möglich ist.

Die hohe Selektivität dieses Gassensors in Bezug auf Wasserstoff und seine hohe Unempfindlichkeit in Bezug auf Ethen wird im Diagramm klar nachgewiesen:

Bei alleiniger Aufgabe von Ethen (10 min ≤ t ≤ 55 min) zeigt sich keine signifikante Änderung des typischen Widerstandsverlaufs am Gassensors, abgegriffen über die Meßstruktur 5.

Dabei ist diese Unempfindlichkeit weitgehend unabhängig von der Konzentration des Ethen.

Bei variierter Aufgabe von H₂ und konstanter Aufgabe von Ethen (ca. 62,5 min ≤ t ≤ 105 min) zeigen sich deutliche Änderungen im Verlauf des elektrischen Widerstands R, welche zeitlich mit der Änderung der am Gassensor anliegenden Konzentration p des H₂ korrelieren.

Dieses Verhalten des Widerstands R ist sehr gut reproduzierbar. Somit ist die Empfindlichkeit des Gassensors in Bezug auf H₂ und seine Unempfindlichkeit auf Ethen nachgewiesen.

Auch ist eine schnelle Reaktion des Sensorsignals aus eine Änderung der Konzentration von H₂ nachgewiesen.

Ein solcher Gassensor ist darüber hinaus preiswert herstellbar, weil er auf einem preiswerten Ga₂O₃-Gassensor basiert und die Schichtaufbringung einfach ist.

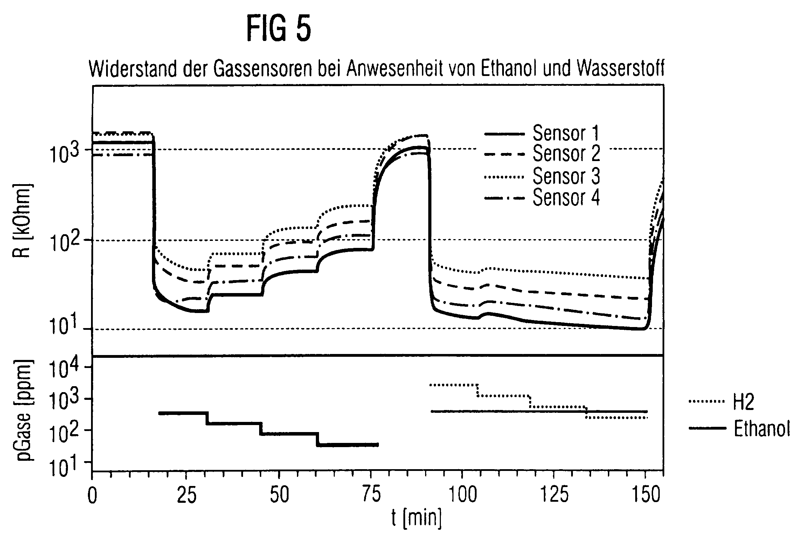

Figur 5 zeigt analog zur Figur 4 den an der Meßstruktur 5 abgegriffenen Widerstand R in kOhm und die am Gassensor anliegende Gaskonzentration p in ppm, jeweils in logarithmischer Skala, aufgetragen gegen die Zeit t in Minuten für einen selektiven Ethanol-Sensor.
Der Widerstand R ist für vier verschiedene Ethanol-Sensoren mit prinzipiell gleichem Aufbau dargestellt. Die Ethanol-Sensoren werden mit Ethanol (durchgehende Linie) und mit Wasserstoff (gepunktete Linie) beaufschlagt.

Der verwendete Gassensor weist als gasaktive Beschichtung 3 eine gesputterte Ga₂O₃-Dünnschicht auf und als Filterschicht 2 eine SiO₂-Schicht. Als Detektionsschicht 1 wird eine hochtemperaturgeheizte halbleitende Metalloxidschicht aus Ga₂O₃ verwendet.

Während einer alleinigen Aufbringung von Ethanol (10 min ≤ t ≤ 75 min) ändert sich der Widerstand R aller vier Sensoren deutlich und weitgehend gleichartig. Die unterschiedliche Höhe des Sensorsignales läßt sich aus herstellungsbedingten Unterschieden erklären.

Aus dem Diagramm wird deutlich, daß ein solcher Ethanol-Sensor erstens hochselektives Verhalten auf Ethanol zeigt, zweitens schnell auf eine Veränderung der Ethanolkonzentration anspricht und gegenüber H₂ weitgehend unempfindlich ist.

Bei einer Beaufschlagung des Gassensors mit einer konstanten Konzentration von Ethanol bei einer variierten Konzentration von H₂ (87,5 min ≤ t ≤ 150 min) zeigt der Widerstand R des Gassensors eine nur sehr geringe Abhängigkeit von der Konzentration des H₂. Diese ist im Vergleich zur Änderung des Widerstands R bei einer Änderung der Ethanolkonzentration vernachlässigbar gering. Somit ist eine geringe Querempfindlichkeit gegenüber H₂ nachgewiesen.

In diesem Ausführungsbeispiel setzt wird das ankommende Ethanol an der oberen gasaktiven Beschichtung 3 aufgespalten ("gecrackt"), wobei langkettige oder größere Kohlenwasserstoffe in mehrere kleine Moleküle ("Crackprodukte") zerlegt werden. So können aus Ethanol Wasserstoffatome abgespalten werden, welche sich störend auf eine Messung auswirken können (siehe auch Figur 4). Die Crackprodukte wechselwirken mit der gasaktiven Beschichtung 3, als Resultat entsteht an der der Filterschicht 2 zugewandten Seite der gasaktiven Beschichtung 3 eine geladene Zone, die durch die (isolierenden SiO₂-) Filterschicht 2 hindurch auf die Detektionsschicht 1 wirkt.

Dadurch ist es möglich, das Ethanol hochselektiv zu detektieren. Das Ethanol reagiert mit dem Ga₂O₃ an der Oberfläche, wobei sich dieses auflädt und mit dem oben beschriebenen Feldeffekt die unter dem SiO₂ liegende, zweite Ga₂O₃-Schicht elektrostatisch beeinflußt.

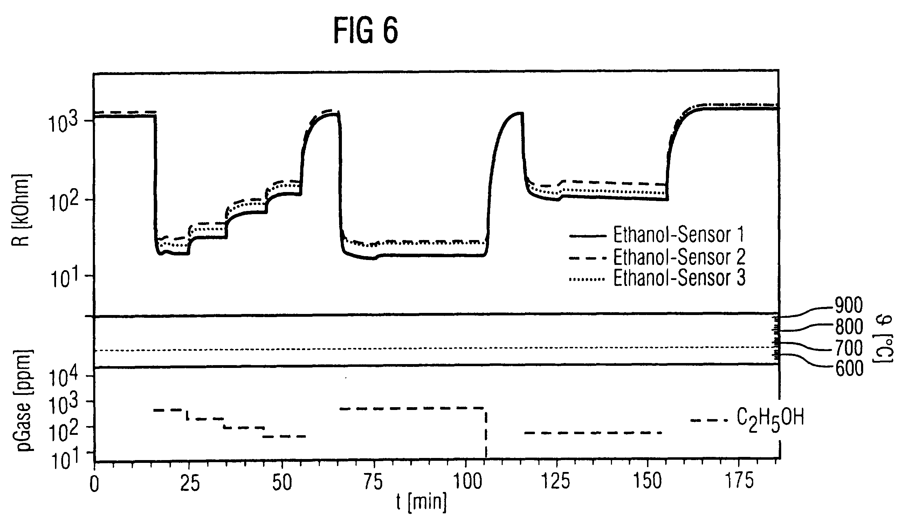

Figur 6 zeigt ein zu den Figuren 4 und 5 analoges Meßdiagramm als Auftragung des an der Meßstruktur 5 abgegriffenen Widerstands R in kOhm gegen die Meßzeit t in min für drei verschiedene Ethanolsensoren gleicher Bauart (oberes Diagramm) sowie für die Konzentration von Ethanol (C₂H₅OH) am Gassensor in ppm, aufgetragen über die gleiche Zeitachse (unteres Diagramm).

Der für diese Messung verwendete Gassensor weist eine gasaktive Beschichtung 3 aus Al₂O₃, eine Filterschicht 2 aus SiO₂ und eine Detektionsschicht 1 aus Ga₂O₃ auf.

Die gasaktive Beschichtung 3 hält bis auf Alkohol alle Gase des Umgebungsgasgemisches, typischerweise: Luft, ab. Die Filterschicht 2 crackt Ethanol und ist für Wasserstoff durchlässig. Die Detektionsschicht 1 und besitzt auch eine Querempfindlichkeit auf Ethanol, welches am gecrackt wird.

Durch diese doppelte Filterwirkung von gasaktiver Beschichtung 3 und Filterschicht 2 werden die Vorteile des einfachen Ga₂O₃-Sensors, wie Langzeitstabilität und Reproduzierbarkeit der Meßergebnisse, voll erhalten. Zusätzlich dazu wird aber durch Einsatz der Filterschicht 2 und der gasaktiven Beschichtung 3 die Selektivität und die Sensitivität des Gassensors verbessert. Weiterhin zeigt sich, daß durch die Auswahl von Al₂O₃ als gasaktiver Beschichtung 3 jede katalytische Wirkung unterbleibt und so keine neuen Querempfindlichkeiten entstehen.

Erst durch den Einsatz der Zwischenschicht 2 aus SiO₂ wird der Einsatz einer Al₂O₃-Schicht in Verbindung mit einem Ga₂O₃-Sensor möglich, weil das Al₂O₃ nicht direkt auf das Ga₂O₃ aufgebracht werden kann.

Dieser Gassensor läßt sich vorteilhaft in einem System zur Prozeßüberwachung einsetzen, zur Überwachung eines Grenzwertes in der Umweltschutztechnik oder zur Kontrolle der Verkehrstauglichkeit einer vermutet alkoholisierten Person. Ein weiteres Einsatzgebiet dieser Kombination von Filterschicht 2 und gasaktiver Beschichtung 3 ist die Korrektur von Ethanol-Querempfindlichkeiten anderer Gassensoren, z. B. eines H₂-Sensors, dessen Einsatzgebiet u. a. eine Branddetektion ist.

Es wird deutlich die hohe Sensitivität und schnelle Ansprechzeit des Gassensors auf Ethanol (C₂H₅OH) gezeigt (10 min ≤ t ≤ 55 min). Man erkennt zudem die gute Langzeit-Stabilität des Sensorsignals (60 min ≤ t ≤ 105 min und 110 min ≤ t ≤ 155 min).

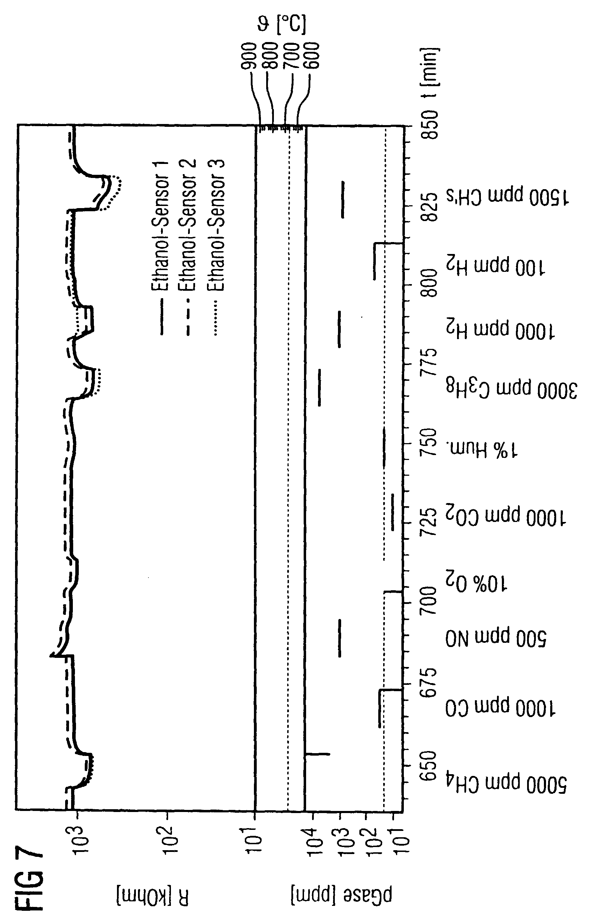

Figur 7 zeigt eine Auftragung des Sensorsignals als Widerstand R in kOhm des in Figur 6 beschriebenen Ethanolsensors für drei verschiedene Ethanolsensoren in Abhängigkeit von der Zeit t in min. Darunter ist auf gleicher Zeitachse die am Gassensor anliegende Konzentration p in ppm für verschiedene Störgase, nämlich 5000 ppm CH₄, 1000 ppm CO, 500 ppm NO, 10% O₂, 1000 ppm CO₂, 1% Hum, 3000 ppm C₃H₈, 1000 ppm H₂, 100 ppm H₂ und 1500 ppm CH's (ein Gemisch verschiedener Kohlenwasserstoffe wie Ethan, Ethen, Propan und Isobuten) dargestellt.

Man erkennt sofort die sehr gute Unterdrückung bezüglich der verschiedenen Störgase. Die größte Querempfindlichkeit liegt um einen Faktor 10 unter dem Nutzsignal.

## Patentansprüche

1. Gassensor, aufweisend
eine gassensitive Detektionsschicht (1),
**dadurch gekennzeichnet**, daß
- auf der Detektionsschicht (1) mindestens eine Filterschicht (2) und
- auf der mindestens einen Filterschicht (2) mindestens eine gasaktive Beschichtung (3) vorhanden ist.

2. Gassensor nach Anspruch 1, bei dem
mehrere Paare einer gasaktiven Beschichtung (3) und einer Filterschicht (2) übereinander angebracht sind.

3. Gassensor nach einem der vorhergehenden Ansprüche, bei dem die Detektionsschicht (1) Ga₂O₃ enthält.

4. Gassensor nach einem der vorhergehenden Ansprüche, bei dem mindestens eine gasaktive Beschichtung (3) ein p-leitendes Material enthält.

5. Gassensor nach Anspruch 4, bei dem
das p-leitende Material Cr-dotiertes SrTiO₃ ist.

6. Gassensor nach einem der vorhergehenden Ansprüche, bei dem die mindestens eine gasaktive Beschichtung (3) eine Ga₂O₃-Dünnschicht ist.

7. Gassensor nach einem der vorhergehenden Ansprüche, bei dem die mindestens eine gasaktive Beschichtung (3) Al₂O₃ enthält.

8. Gassensor nach einem der vorhergehenden Ansprüche, bei dem die mindestens eine gasaktive Beschichtung (3) Goldcluster aufweist.

9. Gassensor nach einem der vorhergehenden Ansprüche, bei dem die mindestens eine Filterschicht (2) SiO₂, Si₃N₄ oder Al₂O₃ enthält.

10. Gassensor nach Anspruch 9, bei dem
die mindestens eine Filterschicht (2) eine SiO₂-Schicht ist.
